# EUROPÄISCHE PATENTSCHRIFT

(11) **EP 1 360 159 B1**
(45) Veröffentlichungstag und Bekanntmachung des Hinweises auf die Patenterteilung: **30.09.2009**
(21) Anmeldenummer: 02708313.8
(22) Anmeldetag: 25.01.2002
(51) Int. Cl.: C07B 43/04, C07B 31/00, C07C 209/32

(54) **VERFAHREN ZUR HYDRIERUNG VON FLÜSSIGEN ORGANISCHEN VERBINDUNGEN**
METHOD FOR HYDROGENATING LIQUID ORGANIC COMPOUNDS
PROCEDE D'HYDROGENATION DE COMPOSES ORGANIQUES LIQUIDES

(30) Priorität: 02.02.2001 DE 10105277
(43) Veröffentlichungstag der Anmeldung: 12.11.2003
(73) Patentinhaber: BASF SE, 67056 Ludwigshafen (DE)
(72) Erfinder: ZEHNER, Peter, 67071 Ludwigshafen (DE); BEY, Oliver, 67150 Niederkirchen (DE); GEORGI, Gunter, Baton Rouge , LA 70817 (US); MÜLLER, Jörn, 49152 Bad Essen (DE)
(86) Internationale Anmeldenummer: PCT/EP2002/000766
(87) Internationale Veröffentlichungsnummer: WO 2002/062729

(56) Entgegenhaltungen:
- EP-A- 0 124 010
- WO-A-87/07598
- WO-A-97/30967
- DE-A- 2 603 076
- DE-C- 4 102 860
- US-A- 4 717 774
- DATABASE CA [Online] CHEMICAL ABSTRACTS SERVICE, COLUMBUS, OHIO, US; retrieved from STN Database accession no. 120:191313/DN, HCAPLUS XP002203078 & HU 63 606 A (NITROIL VEGYIPAR TERMELO-FEJLESZTO RT.) 28. September 1993 (1993-09-28)
- DATABASE WPI Section Ch, Week 199807 Derwent Publications Ltd., London, GB; Class B03, AN 1998-075478 XP002203079 & RU 2 081 111 C (BORESKOVA INST. KATALIZA SIBIR; NOVOKUZNETSKIJ NI KHIM FARMATS), 10. Juni 1997 (1997-06-10)

## Beschreibung

Die Erfindung betrifft ein Verfahren zur Hydrierung von flüssigen organischen Verbindungen, insbesondere zur Hydrierung von Nitroverbindungen zu Aminen.

Die Hydrierung von flüssigen organischen Verbindungen ist eines der häufigsten Verfahren in der Chemieindustrie. Eine technisch bedeutende Hydrierreaktion ist die Hydrierung von Nitroverbindungen zu den entsprechenden Aminen.

Bei dieser Art von Hydrierungen hat die Hydrierung von Nitroaromaten zu aromatischen Aminen eine besondere Bedeutung. Aromatische Amine können vielfach eingesetzt werden. So ist das Anilin Ausgangsprodukt für viele organische Synthesen. Toluylendiamin, häufig auch als TDA bezeichnet, kann mit Phosgen zu Toluylendiisocyanat, einem der am häufigsten für die Herstellung von Polyurethanen eingesetzten Isocyanate, umgesetzt werden.

Die katalytische Hydrierung von flüssigen Einsatzstoffen erfolgt in der Technik überwiegend mit einem fein suspendierten Katalysator oder im Festbett. Als Reaktoren können Autoklaven, Schlaufenreaktoren, Rührkessel, Blasensäulen oder Reaktorkaskaden eingesetzt werden. Derartige Verfahren werden beispielsweise in EP-A-263 935, EP-A-634 391, EP-A-124 010 oder WO 00/35852 beschrieben.

Da die Löslichkeit des Wasserstoffs im allgemeinen in der flüssigen Phase sehr gering ist, werden die in der Technik bekannten Verfahren unter Druck betrieben, um so die Sättigungskonzentrationen des Wasserstoffs zu erhöhen. Der gasförmige Wasserstoff wird häufig am Boden der Reaktoren eingeleitet und durch Rührer, Düsen oder bei der Durchströmung von Packungen dispergiert, um eine hohe spezifische Austauschfläche zwischen der gasförmigen und der flüssigen Phase zu erreichen.

Nachteilig ist allerdings, dass das System aus Hydrierbad und Wasserstoff stark koalesziert, d.h. dass unmittelbar nach Verlassen der Dispergierzone wieder verhältnismäßig große Blasen gebildet werden, die die spezifischen Austauschflächen und somit die Stofftransportleistung deutlich verringern. Dieses Verhalten steht im Gegensatz zu der bislang vorherrschenden Meinung, dass ein Vielstoffgemisch, wie das Hydrierbad, überwiegend koaleszenzgehemmt sei und die Eigenschaften des Gases einen vernachlässigbaren Einfluss auf das Koaleszenzverhalten haben.

Der für die Hydrierungen eingesetzte Wasserstoff, der in der Technik üblicherweise in Synthesegasanlagen hergestellt wird, weist zumeist eine Reinheit von über 99,95 Vol.-% Wasserstoff auf. Damit soll erreicht werden, dass möglichst wenig Inertgase in die Hydrierreaktoren eingeschleust werden.

Vorteilhaft bei der Fahrweise mit sehr reinem Wasserstoff ist der stets hohe Wasserstoffpartialdruck in der Gasphase des Reaktors, der den Übergang von der Gasphase in das flüssige Hydrierbad erleichtert.

Weiterhin kann dadurch erreicht werden, dass nur eine geringe Menge des dem Reaktor zugeführten Wasserstoffs als Abgas aus dem Reaktor ausgeschleust werden muss.

Zumeist werden bei den technisch üblichen Verfahren ca. 1 bis 2 % des in den Reaktor gefahrenen Wasserstoffs als Abgas ausgeschleust, damit die gasförmigen Nebenprodukte und die geringen Anteile an Inerten aus dem Reaktionsgemisch entfernt werden. Dies führt jedoch in der Regel dazu, dass die Reinheit an Wasserstoff im Abgasstrom nicht unter 98 Vol.-% sinkt.

Bei den Verfahren des Standes der Technik kann auf Grund der oben beschriebenen Koaleszenz bei der hohen Reinheit des Wasserstoffs der erhöhte Partialdruck im Reaktor nicht ausgenutzt werden. Daher kommt es auf Grund der mangelnden Austauschfläche zwischen flüssiger und gasförmiger Phase zu einer erhöhten Alterung des Katalysators sowie einer unzureichenden Selektivität der Reaktion, da die Sättigungskonzentrationen des Wasserstoffs im Hydrierbad nicht erreicht werden können.

Aufgabe der Erfindung war es, ein Verfahren zur Hydrierung von flüssigen organischen Verbindungen, insbesondere zur Hydrierung von Nitroverbindungen zu Aminen, zu entwickeln, bei dem ein optimaler Stoffaustausch zwischen dem Wasserstoff und dem Hydrierbad stattfindet, die Alterung des Katalysators unterdrückt sowie die Selektivität der Umsetzung erhöht wird.

Überraschenderweise wurde gefunden, dass bei einer Mischung des für die Hydrierung eingesetzten Wasserstoffs mit Anteilen mindestens eines bei der Hydrierreaktion inerten Gases eine stärkere Koaleszenzhemmung vorliegt und die Stofftransportleistung des Hydrierreaktors deutlich erhöht werden kann.

Gegenstand der Erfindung ist demzufolge ein Verfahren zur Hydrierung von flüssigen organischen Verbindungen, insbesondere zur Herstellung von Aminen durch Hydrierung der entsprechenden Nitroverbindungen, dadurch gekennzeichnet, dass der eingesetzte Wasserstoff Anteile mindestens eines bei der Hydrierreaktion inerten Gases enthält.

Der Anteil des bei der Hydrierreaktion inerten Gases in der Gasphase der Reaktion liegt vorzugsweise im Bereich zwischen 3 und 50 Vol.-%, bezogen auf die Gasmenge im Reaktionsgemisch. Prinzipiell kann auch außerhalb dieser Grenzen gearbeitet werden. Bei Gehalten unterhalb dieses Bereiches kommt es allerdings noch immer zu einer störenden Koaleszenz des Gases in der Flüssigphase. Bei Gehalten oberhalb dieses Bereiches kann auf Grund des geringen Partialdrucks des Wasserstoffs trotz erhöhter Austauschfläche die Konzentration an gelöstem Wasserstoff im Hydrierbad sinken, wodurch die Ausbeute der Reaktion abfällt. Vorzugsweise liegt der Gehalt an inerten Gasen im Bereich zwischen 3 und 30 Vol.-%, besonders bevorzugt im Bereich zwischen 5 und 20 Vol.-%, jeweils bezogen auf die Gasmenge im Reaktionsgemisch.

Die bei der Hydrierreaktion inerten Gase müssen schwerer sein als Wasserstoff. Prinzipiell sind für das erfindungsgemäße Verfahren alle Stoffe einsetzbar, die unter den Bedingungen der Hydrierung gasförmig sind und sich bei der Reaktion inert verhalten. Beispiele für mögliche Gase sind Stickstoff, Edelgase, insbesondere Neon, Argon, Krypton, Ammoniak, niedere gesättigte Kohlenwasserstoffe, insbesondere Methan, Ethan, Propan, Butan, oder Kohlendioxid. Die größte Bedeutung hat hierbei der Stickstoff, da dieser unproblematisch und sicher zu handhaben ist und in großtechnischen Anlagen zumeist ausreichend zur Verfügung steht. Unter bestimmten Reaktionsbedingungen kann auch Wasserdampf als bei der Hydrierreaktion inertes Gas wirken. Das Wasser entsteht teilweise bei der Hydrierung, es kann aber auch mit den Reaktionskomponenten oder als Lösungsmittel in den Reaktor gelangen. Prinzipiell ist jedoch solchen inerten Stoffen der Vorzug zu geben, die unter den im Reaktor herrschenden Bedingungen nicht kondensieren können, um Druckschwankungen oder schwankende Zusammensetzungen der Gasphase zu vermeiden.

Die bei der Hydrierreaktion inerten Gase können im Gemisch mit dem Wasserstoff dem Reaktor zugeführt werden. Es ist jedoch auch möglich, diese in einem separaten Strom dem Reaktor zuzuführen.

In einer weiteren Ausführungsform der Erfindung wird die Konzentration der bei der Hydrierreaktion inerten Gase allein durch die Regelung des Abgasstroms eingestellt. Durch eine Drosselung des Abgasstroms aus dem Reaktor kann die Konzentration der bei der Hydrierreaktion inerten Gase im Reaktor innerhalb des bevorzugten Bereichs eingestellt werden.

Die allgemein im Einsatz befindlichen Hydrierreaktoren wie Autoklaven, Blasensäulen, Rührkessel, Schlaufen- oder Festbettreaktoren sind durch gezielte Gasrückführungen oder durch das Wiedereinschlagen (Redispergieren) von Gas aus der Gasphase durch Treibstrahlen oder Rührer gasseitig im allgemeinen gut rückvermischt. Daher wird der Abgasstrom, der in der Regel direkt aus der Gasphase des Reaktors entnommen wird, zumeist die gleiche Zusammensetzung haben wie das im Reaktor zirkulierende Gas selbst.

Aufgrund der im wesentlichen vollständigen Rückvermischung der Gasphase im Reaktor kann durch Vorgeben der Abgasmenge bei dieser Ausführungsform des erfindungsgemäßen Verfahrens die gewünschte Konzentration des Wasserstoffs im Reaktor eingestellt werden. Beträgt die Konzentration des frischen Wasserstoffs 99,95 Vol.-%, so wird bei einer Ausschleusungsrate von 1 %, bezogen auf das Frischgas, der Anteil an inerten Gasen in der Gasphase des Reaktors bei mindestens 5 Vol.-%, bezogen auf die Gasphase des Reaktors, liegen. Bei einer Verringerung der Ausschleusung auf 0,25 %, bezogen auf das Frischgas, können sogar mindestens 20 Vol.-% inerte Gase im Reaktor akkumuliert werden. Noch wesentlich höhere Anteile an inerten Gasen sind allerdings, wie oben ausgeführt, aufgrund einer zu starken Absenkung des Wasserstoffpartialdruckes nicht mehr sinnvoll.

Daher sollte bei der Regelung der Konzentration des Wasserstoffs in der Reaktionsmischung über die Einstellung der Abgasmenge die untere Grenze der Konzentration des zugeführten Wasserstoffs bei 98 Vol.-% liegen. In diesem Fall kann bei einer Ausschleusrate von 10 %, bezogen auf das Frischgas, ein Inertgasanteil im Reaktor von ca. 20 Vol.-% eingestellt werden. Bei niedrigeren Ausschleusraten steigt der Inertgasanteil über die besonders bevorzugten Werte, bei höheren Ausschleusraten kann das Verfahren unwirtschaftlich werden, da zu große Mengen Wasserstoff als Abgas abgeführt werden müssen. Prinzipiell kann auch bei Reinheiten des frischen Wasserstoffs von über 99,95 Vol.-% der erfindungsgemäße Inertgasanteil in der Gasphase des Reaktors eingestellt werden. Allerdings muss die Abgasmenge bzw. die Ausschleusrate dann stark reduziert werden. Die Regelung derartig kleiner Abgasströme kann in der Praxis problematisch werden. Auf diese Weise kann aber bei einer beispielhaften Reinheit des frischen Wasserstoffs von 99,99 Vol.-% durch Reduzierung des Ausschleusrate auf 0,2 % ein Inertgasanteil in der Gasphase des Reaktors von 5 Vol.-% eingestellt werden.

Prinzipiell ist es auch möglich, zum Erreichen der gewünschten Konzentration des Wasserstoffs das Abgasventil völlig zu schließen und nur bei Überschreitung eines bestimmten Inertgasanteils diskontinuierlich zu öffnen.

Besonders vorteilhaft ist das erfindungsgemäße Verfahren bei Hydrierungen, bei denen Reaktionswasser anfällt und die bei Temperaturen unter 150°C betrieben werden.

Der Vorteil einer Hydrierung bei Temperaturen oberhalb 150°C besteht in einem größeren Dampfdruck des bei der Hydrierung anfallenden Reaktionswassers, welches dann ebenfalls als ein bei der Hydrierreaktion inertes Gas wirkt, wodurch das Anreichern von anderen Inerten im Reaktor reduziert werden kann beziehungsweise nicht mehr nötig ist. Allerdings sind mit einer Hydrierung bei über 150°C Nachteile verbunden, beispielsweise eine beschleunigte Katalysatoralterung und eine erhöhte Nebenproduktbildung. Daher sind Temperaturen über 150°C für das erfindungsgemäße Verfahren nicht bevorzugt.

Die Zuführung des frischen Wasserstoffs kann in die Gasphase des Reaktors erfolgen. Dies ist insbesondere bei Strömungsreaktoren, wie sie beispielsweise in EP 634 391 oder WO 00/35852 beschrieben sind, vorteilhaft. In diesem Fall muss jedoch ausgeschlossen werden, dass es zu einem Kurzschluss mit der Abgasleitung kommt.

Vorzugsweise wird der frische Wasserstoff in die Flüssigphase des Reaktors dosiert. Die Einleitung kann mit bekannten Dosierorganen erfolgen. Möglichkeiten sind beispielsweise die Einleitung über eine oder mehrere Ringleitungen in der Flüssigphase, ein oder mehrere Einleitrohre in der Flüssigphase, insbesondere am Boden des Reaktors, oder bei Rührkesseln über einen Hohlwellenrührer.

Das Abgas wird zumeist am Kopf des Reaktors über die Gasphase abgezogen. Wie bereits ausgeführt, muss sichergestellt sein, dass bei der Entfernung des Abgases kein Kurzschluss zum frischen Wasserstoff auftritt, um zusätzliche Verluste an Wasserstoff zu vermeiden.

Das erfindungsgemäße Verfahren kann prinzipiell bei allen Hydrierungen von organischen Verbindungen, die bei den Reaktionsbedingungen flüssig sind, angewendet werden. Beispiele hierfür sind die Hydrierung von Benzol zum Cyclohexan, von Butindiol zum Butandiol und der Oxoaldehyde zu den Oxoalkoholen. Bei diesen Verfahren entsteht jeweils kein Reaktionswasser, was die Anwesenheit von inerten Gasen besonders wichtig macht. Besonders vorteilhaft kann das erfindungsgemäße Verfahren bei der Herstellung von Aminen aus den entsprechenden Nitroverbindungen, insbesondere von aromatischen Aminen aus den entsprechenden aromatischen Nitroverbindungen, angewendet werden.

Vorzugsweise werden im Rahmen des erfindungsgemäßen Verfahrens aromatische Nitroverbindungen mit einer oder mehreren Nitrogruppen und 6 bis 18 Kohlenstoffatomen, beispielsweise Nitrobenzole, wie o-, m-, p-Nitrobenzol, 1,3-Dinitrobenzol, Nitrotoluole, wie z.B. 2,4-, 2,6-Dinitrotoluol, 2,4,6-Trinitrotoluol, Nitroxylole, wie z.B. 1,2-Dimethyl-3-, 1,2 Dimethyl-4-, 1,4-Dimethyl-2-, 1,3-Dimethyl-2-, 2,4-Dimethyl-1- und 1,3-Dimethyl-5-nitrobenzol, Nitronaphthaline, wie z.B. 1-, 2-Nitronaphthalin, 1,5 und 1,8-Dinitronaphthalin, Chlornitrobenzole, wie z.B. 2-Chlor-1,3-, 1-Chlor-2,4-dinitrobenzol, o-, m-, p-Chlornitrobenzol, 1,2-Dichlor-4-, 1,4-Dichlor-2-, 2,4-Dichlor-1-und 1,2-Dichlor-3-nitrobenzol, Chlornitrotoluole, wie z.B. 4-Chlor-2, 4-Chlor-3-, 2-Chlor-4- und 2-Chlor-6-nitrotoluol, Nitroaniline, wie z.B. o-, m-, p- Nitroanilin; Nitroalkohole, wie z.B. Tris(hydroxymethyl)-nitromethan, 2-Nitro-2-methyl-, 2-Nitro-2-ethyl-1,3-propandiol, 2-Nitro-1-butanol und 2-Nitro-2-methyl-1-propanol sowie beliebige Gemische aus zwei oder mehreren der genannten Nitroverbindungen eingesetzt.

Bevorzugt werden nach dem erfindungsgemäßen Verfahren aromatische Nitroverbindungen, vorzugsweise Mononitrobenzol, Methylnitrobenzol oder Methylnitrotoluol, und insbesondere 2,4-Dinitrotoluol oder dessen technische Gemische mit 2,6-Dinitrotoluol, wobei diese Gemische vorzugsweise bis zu 35 Gewichtsprozent, bezogen auf das Gesamtgemisch, an 2,6-Dinitrotoluol mit Anteilen von 1 bis 4 Prozent an vicinalem Dinitrotoluol und 0,5 bis 1,5 % an 2,5- und 3,5-Dinitrotoluol aufweisen, zu den entsprechenden Aminen hydriert. Insbesondere bei der Hydrierung von Dinitrotoluolisomeren zu den entsprechenden Toluylendiaminderivaten (TDA) kann das erfindungsgemäße Verfahren vorteilhaft eingesetzt werden.

Die Hydrierung der aromatischen Amine kann in Substanz oder in Lösung durchgeführt werden. Als Lösungsmittel werden die dafür üblichen Stoffe, insbesondere niedere Alkohole, vorzugsweise Ethanol, eingesetzt.

Die erfindungsgemäße Hydrierung wird üblicherweise in Gegenwart von Katalysatoren durchgeführt. Als Katalysatoren können die üblichen und bekannten Hydrierkatalysatoren eingesetzt werden.

Beispiele hierfür sind Metalle der VIII. Nebengruppe des Periodensystems, die auf Trägermaterialien wie Aktivkohle oder Oxiden des Aluminiums, des Siliciums oder anderer Materialien aufgebracht sein können. Vorzugsweise werden Raney-Nickel und/oder geträgerte Katalysatoren auf Basis von Nickel, Palladium und/oder Platin verwendet. Prinzipiell möglich ist auch der Einsatz von homogenen Katalysatoren.

Das erfindungsgemäße Verfahren unter Verwendung von heterogenen Katalysatoren kann in Festbett- oder Suspensionsfahrweise durchgeführt werden. Die Festbettfahrweise kann dabei in Sumpf- oder in Rieselfahrweise durchgeführt werden.

Bei der Suspensionsfahrweise werden ebenfalls heterogene Katalysatoren eingesetzt. Die bevorzugte Hydrierung von Nitroverbindungen zu Aminen wird ebenfalls zumeist in Anwesenheit von heterogenen Katalysatoren durchgeführt. Die heterogenen Katalysatoren werden dabei zumeist in feinverteiltem Zustand eingesetzt und liegen in der Reaktionssuspension feinteilig suspendiert vor. Als Reaktoren bei der Hydrierung in Suspension kommen insbesondere Schlaufenapparate, wie Strahlschlaufen oder Propellerschlaufen, Rührkessel, die auch als Rührkesselkaskaden ausgestaltet sein können, Blasensäulen oder Air-Lift-Reaktoren zum Einsatz.

Das erfindungsgemäße Verfahren wird zumeist bei den für die spezielle Reaktion üblichen Reaktionsbedingungen durchgeführt. So wird die Umsetzung von aromatischen Nitroverbindungen zu aromatischen Aminen üblicherweise bei einem Druck im Bereich von 5 bis 100 bar, bevorzugt 10 bis 50 bar, und einer Temperatur von 80 bis 160°C, bevorzugt 80 bis 150°C und insbesondere 100 bis 150°C durchgeführt.

Überraschenderweise gelingt es bei dem erfindungsgemäßen Verfahren, die Austauschfläche zwischen Gasphase und Flüssigphase deutlich zu erhöhen. Die höhere Austauschfläche ermöglicht, stets dicht an der Sättigungskonzentration des Wasserstoffs im Hydrierbad zu arbeiten. Dadurch erhöht sich die Wahrscheinlichkeit, dass die freien Plätze an der Katalysatoroberfläche von Wasserstoffmolekülen belegt werden. Das führt in der Folge zu einer Erhöhung der Ausbeute und der Selektivität des Verfahrens.

Das erfindungsgemäße Verfahren kann problemlos und ohne zusätzliche Umbauten in allen bestehenden Hydrierreaktoren eingesetzt werden. Die für das erfindungsgemäße Verfahren bevorzugten inerten Gase, insbesondere der Stickstoff, stehen praktisch an allen Produktionsstandorten ausreichend zur Verfügung.

Ein weiterer Vorteil des erfindungsgemäßen Verfahrens besteht darin, dass eine aufwendige Reinigung des eingesetzten Wasserstoffs nicht mehr erforderlich ist. Es genügt, solche Bestandteile, die als Katalysatorgift wirken beziehungsweise zu Nebenreaktionen führen können, aus dem Wasserstoff zu entfernen. Damit kann die Wasserstofferzeugung kostengünstiger gestaltet werden.

Die Erfindung soll an den nachfolgenden Beispielen näher erläutert werden.

### Beispiel 1 (Vergleich)

Es wurde ein zylinderförmiger Reaktor mit einem außenliegenden Kreislauf, einer Prallplatte im unteren Reaktorteil sowie einem konzentrischen Einsteckrohr eingesetzt, wie er in WO 00/35852 in Beispiel 1 beschrieben ist. Das Reaktionsvolumen des Reaktors betrug 0,05 m³. Der Reaktor war mit 36 parallel geschalteten Fieldrohren versehen, die insgesamt einer Kühlfläche von etwa 2,5 m² entsprachen. Die Menge des in die Fieldrohre eingespeisten Kühlwassers betrug 1 m³/h, die Temperatur des in die Fieldrohre eingespeisten Kühlwassers lag bei 30°C.

Mit einer Hochdruckpumpe wurden 40,3 kg/h einer Dinitrotoluolschmelze, bestehend aus 80 Gewichtsteilen 2,4-Dinitrotoluol und 20 Teilen 2,6-Dinitrotoluol, bei 120°C in ein schnell fließendes Gemisch aus ca. 62 Gewichtsteilen eines entsprechenden Diaminotoluolgemisches, 36 Gewichtsteilen Wasser und 2 Gewichtsteilen eines feinverteilten Ni-Hydrierkatalysators eingedüst. Durch gleichzeitiges Einleiten von 30 Nm³/h Wasserstoff wurde im Reaktor ein Druck von 25 bar aufrechterhalten. Zur Aufrechterhaltung der Schlaufenströmung wurde im externen Produktkreislauf ein Volumenstrom von 2,6 m³/h umgewälzt. In der Reaktionsdüse herrschte ein Druck von rund 3 bar, der Leistungseintrag betrug 5 kW/m³. Die Reaktion verlief unter nahezu isothermen Bedingungen, da die entstehende Reaktionswärme bereits am Ort ihrer Entstehung abgeführt wurde. Die maximale Reaktionstemperatur im unteren Drittel des Reaktors betrug 122°C. Dem Reaktor wurden gleichzeitig unter Rückhaltung des Katalysators 26,7 kg/h eines entsprechenden Diaminotoluolgemisches sowie 15,8 kg/h Wasser kontinuierlich entnommen, was einer Raum-Zeit-Ausbeute von 580 kg Amingemisch/m³*h entsprach.

Die Reinheit des zugeführten Wasserstoffs betrug 99,99999 Vol.-% bei einer Ausschleusrate von ca. 1 bis 2 %. Das Abgas wurde mit Hilfe eines Kondensators von den kondensierbaren Anteilen, die im wesentlichen Wasserdampf enthielten, getrennt und mit einer Reinheit von über 99,5 Vol.-% Wasserstoff zur Fackel geführt. Es fand keine Zuführung zusätzlicher Inertgase statt. Die Hydrierung wurde bei 120°C und 26 bar Absolutdruck betrieben. Der Gewichtsanteil an Wasser im Hydrierbad betrug ca. 35 %.

Die Gasphase im Reaktor setzte sich nahezu ausschließlich aus dem Wasserdampf des Reaktionswassers und dem Wasserstoff zusammen. Rechnerisch lässt sich somit eine Gesamtdichte der Gasphase von ca. 2,5 kg/m³ ermitteln. Bei der Begasung des Reaktors mit Hilfe der Düse und des internen Umlaufs konnten Gasgehalte von maximal 10 Vol.-%, bezogen auf den Volumenanteil des Gases im Hydrierbad in der Flüssigphase des Reaktors eingestellt werden. Höhere Gasgehalte waren nicht zu erreichen. Durch eine Leistungsbilanz des Reaktors ließ sich aus den Messungen ermitteln, dass der mittlere Blasendurchmesser in der Flüssigphase des Reaktors bei ca. 7 mm lag.

### Beispiel 2

Es wurde verfahren wie in Beispiel 1, jedoch wurden zusätzlich 0,1 m³ Stickstoff pro 100 m³ Wasserstoff über eine Einperlleitung zugeführt. Die Abgasmenge des Reaktors wurde so reguliert, dass die Reinheit des ausgeschleusten Wasserstoffs nach Abtrennung des Wasserdampfes durch Kondensation nur noch 90 Vol.-% betrug. Am Gesamtdruck, der Reaktortemperatur und am Gewichtsanteil des Wassers im Hydrierbad fanden keine Veränderungen im Vergleich zu Beispiel 1 statt. Unter der Annahme einer vollständig rückvermischten Gasphase ergab sich somit eine Gesamtdichte des Gases im Reaktor von ca. 4,4 kg/m³, die sich aus der Dichte des Stickstoffs, des Wasserstoffs und des Wasserdampfes zusammensetzt. Messungen haben daraufhin ergeben, dass der Gasgehalt im Hydrierbad des Reaktors ohne weitere Veränderungen der Fahrweise des Reaktors auf 18 Vol.-% bezogen auf den Volumenanteil des Gases im Hydrierbad, anstieg. Dies lässt sich nur auf eine Verringerung der Blasengrößen zurückführen. Eine Leistungsbilanz des Reaktors ergab, dass der mittlere Durchmesser der Gasblasen im Hydrierbad nach der Zugabe des Stickstoffs auf ca. 4 mm abgesunken ist.

### Beispiel 3

Es wurde ein Reaktor wie in Beispiel 1 verwendet. Der frische Wasserstoff wurde mit einer Reinheit von 99,99 Vol.-% in den Reaktor geführt. Bei einer stündlich zugeführte Menge von insgesamt 30 Nm³ Wasserstoff wurden somit tatsächlich 29,997 Nm³/h Wasserstoff und 0,003 Nm³/h inerte Gase eingeschleust. Um eine Wasserstoffreinheit in der Gasphase des Hydrierreaktors von mindestens 99 Vol.-% zu gewährleisten, musste 1% Abgas, bezogen auf die Frischgasmenge, ausgeschleust werden. Das Abgas enthielt dann 0,297 Nm³ Wasserstoff und 0,003 Nm³ Inerte.

Ausgehend von diesem Betriebszustand wurde die Reinheit des frischen Wasserstoffs auf 99,9 Vol.-% reduziert. Dadurch sinkt der Aufwand für die Erzeugung des Wasserstoffs erheblich. Wird nach wie vor 1 % Abgas (bezogen auf die Frischgasmenge) ausgeschleust, d.h. 0,3 Nm³/h verlassen den Reaktor, so hat dieses Abgas die Zusammensetzung von 0,27 m³ H₂ und 0,03 m³ Inerte hat. Somit liegt die Wasserstoffreinheit des Abgases nach Abtrennung der kondensierbaren Anteile bei ca. 90 Vol.-%, was in dem besonders bevorzugten Bereich liegt.

Der Vorteil dieser Verfahrensvariante liegt in einem geringeren Aufwand bei der Reinigung des frischen Wasserstoffs; in einem etwas geringen Verlust an Wasserstoff; sowie kleineren Blasen im Reaktor, die eine größere Austauschfläche bewirken.

### Beispiel 4

Es wurde verfahren wie im Ausgangszustand von Beispiel 3, jedoch wurde die Abgasmenge von 1 % auf 0,1 % bezogen auf die Frischgasmenge, abgesenkt und nicht die Zusammensetzung des frischen Wasserstoffs verändert. So wurden absolut nur noch 0,03 m³/h Abgas ausgeschleust, die sich aus 0,027 m³/h Wasserstoff und 0,003 m³/h Inerte zusammensetzen. Die Konzentration des Wasserstoffs im Abgas des Hydrierreaktors betrug 90 %.

Die Vorteile dieser Variante sind ein deutlich geringerer Wasserstoffverlust und die größere Stoffaustauschfläche aufgrund kleinerer Blasen. Hierzu sind am Reaktor keinerlei weitere Änderungen nötig, das Abgasventil muss lediglich weiter geschlossen werden.

## Patentansprüche

1. Verfahren zur Hydrierung von flüssigen organischen Verbindungen, ausgewählt aus der Gruppe, enthaltend Mononitrobenzol, Methylnitrobenzol, Methylnitrotoluol, 2,4-Dinitrotoluol und Gemische aus 2,4-Dinitrotoluol und 2,6-Dinitrololuol, **dadurch gekennzeichnet, dass** der im Reaktor vorliegende Wasserstoff Anteile mindestens eines bei der Hydrierreaktion inerten Gases, ausgewählt aus der Gruppe, enthaltend Stickstoff, Edelgase und Ammoniak, enthält.

2. Verfahren nach Anspruch 1, **dadurch gekennzeichnet, dass** die Summe der Anteile des bei der Hydrierreaktion inerten Gase im Bereich zwischen 3 und 50 Vol.-%, bezogen auf die Gasphase im Reaktor, beträgt.

3. Verfahren nach Anspruch 1 oder 2, **dadurch gekennzeichnet, dass** die Summe der Anteile der bei der Hydrierreaktion inerten Gase im Bereich 5 und 20 Vol.-%, bezogen auf die Gasphase im Reaktor, beträgt.

4. Verfahren nach einem der Ansprüche 1 bis 3, **dadurch gekennzeichnet, dass** das bei der Hydrierreaktion inerte Gas im Gemisch mit dem Wasserstoff dem Reaktor zugeführt wird.

5. Verfahren nach einem der Ansprüche 1 bis 3, **dadurch gekennzeichnet, dass** das bei der Hydrierreaktion inerte Gas dem Reaktor an einer anderen Stelle als der Wasserstoff zugeführt wird.

6. Verfahren nach einem der Ansprüche 1 bis 4, **dadurch gekennzeichnet, dass** der Gehalt des bei der Hydrierreaktion inerten Gases durch die Regelung der Menge des aus dem Reaktor ausgeschleusten Abgases eingestellt wird.

## Claims

1. A process for hydrogenating liquid organic compounds selected from the group consisting of mononitrobenzene, methylnitrobenzene, methylnitrotoluene, 2,4-dinitrotoluene and mixtures of 2,4-dinitrotoluene and 2,6-dinitrotoluene, wherein the hydrogen present in the reactor comprises proportions of at least one gas which is inert in the hydrogenation reaction and is selected from the group consisting of nitrogen, noble gases and ammonia.

2. The process according to claim 1, wherein the sum of the proportions of the gases which are inert in the hydrogenation reaction is from 3 to 50% by volume, based on the gas phase in the reactor.

3. The process according to claim 1 or 2, wherein the sum of the proportions of the gases which are inert in the hydrogenation reaction is from 5 to 20% by volume, based on the gas phase in the reactor.

4. The process according to any of claims 1 to 3, wherein the gas which is inert in the hydrogenation reaction is fed to the reactor as a mixture with the hydrogen.

5. The process according to any of claims 1 to 3, wherein the gas which is inert in the hydrogenation reaction is fed into the reactor at a different point from the hydrogen.

6. The process according to any of claims 1 to 4, wherein the content of the gas which is inert in the hydrogenation reaction is established by regulating the amount of waste gas discharged from the reactor.

## Revendications

1. Procédé d'hydrogénation de composés organiques liquides choisis dans le groupe contenant le mononitrobenzène, le méthylnitrobenzène, le méthylnitrotoluène, le 2,4-dinitrotoluène et les mélanges de 2,4-dinitrotoluène et de 2,6-dinitrotoluène, **caractérisé en ce que** l'hydrogène présent dans le réacteur contient des fractions d'au moins un gaz inerte au regard de la réaction d'hydrogénation, choisi dans le groupe contenant l'azote, les gaz nobles et l'ammoniac.

2. Procédé selon la revendication 1, **caractérisé en ce que** la somme des fractions des gaz inertes au regard de la réaction d'hydrogénation est dans la plage allant de 3 à 50 % en volume par rapport à la phase gazeuse dans le réacteur.

3. Procédé selon la revendication 1 ou 2, **caractérisé en ce que** la somme des fractions des gaz inertes au regard de la réaction d'hydrogénation est dans la plage allant de 5 à 20 % en volume par rapport à la phase gazeuse dans le réacteur.

4. Procédé selon l'une quelconque des revendications 1 à 3, **caractérisé en ce que** le gaz inerte au regard de la réaction d'hydrogénation est introduit dans le réacteur en mélange avec l'hydrogène.

5. Procédé selon l'une quelconque des revendications 1 à 3, **caractérisé en ce que** le gaz inerte au regard de la réaction d'hydrogénation est introduit dans le réacteur à un autre emplacement que l'hydrogène.

6. Procédé selon l'une quelconque des revendications 1 à 4, **caractérisé en ce que** la teneur en gaz inerte au regard de la réaction d'hydrogénation est ajustée par réglage de la quantité de gaz d'échappement déchargé du réacteur.
